(19) **Europäisches Patentamt**

**European Patent Office**

**Office européen des brevets**

(11) Veröffentlichungsnummer: **0 489 297 A1**

(12) # EUROPÄISCHE PATENTANMELDUNG

(21) Anmeldenummer: **91119684.8**

(22) Anmeldetag: **19.11.91**

(51) Int. Cl.5: **A61M 39/00, A61M 5/168**

(30) Priorität: **04.12.90 DE 4038547**

(43) Veröffentlichungstag der Anmeldung:
**10.06.92 Patentblatt 92/24**

(84) Benannte Vertragsstaaten:
**AT BE CH DE FR GB LI LU NL SE**

(71) Anmelder: **Mokros, Klaus, Dipl.-Ing.**
**Brentanostrasse 51**
**W-8755 Alzenau(DE)**

(72) Erfinder: **Mokros, Klaus, Dipl.-Ing.**
**Brentanostrasse 51**
**W-8755 Alzenau(DE)**

(74) Vertreter: **Ouermann, Helmut, Dipl.-Ing.**
**Postfach 6145 Gustav-Freytag-Strasse 25**
**W-6200 Wiesbaden(DE)**

(54) **Infusionsanordnung mit einer Einrichtung zum Abführen eines Bolusvolumens.**

(57) Die Erfindung betrifft eine Infusionsanordnung mit einer Einrichtung zum Abführen eines Bolusvolumens. Unter Bolusvolumen versteht man ein unerwünschtes Infusionsvolumen, das bei einem patientenseitigen Teilverschluß oder Verschluß durch fortlaufende Infusionszufuhr, insbesondere mittels einer Infusionspumpe, anfällt und bei einer schlagartigen Verabreichung des Patienten diesen erheblich gefährden kann.

Um das Bolusvolumen automatisch abführen und auffangen zu können, schlägt die Erfindung ein Behältnis mit einem pumpenseitigen Zugang (14) und einem patientenseitigen Abgang (5) vor. Innerhalb des Behältnisses (1) ist ein Verschiebekolben (8) verschiebbar gelagert, dieser weist eine Durchströmbohrung (20) auf, die einen pumpenseitigen Druckraum (21) mit einem patientenseitigen Druckraum (22) verbindet. Im gezeigten Normalfall durchströmt die Infusionsflüssigkeit die Durchströmbohrung (20) des Verschiebekolbens (8). Im Bolusfall wird wegen der Druckdifferenz vom pumpenseitigem Druckraum (21) zu patientenseitigem Druckraum (22) der Verschiebekolben (8) in Richtung des patientenseitigen Anschlusses (5) bewegt und gibt damit einen weiteren, seitlichen Anschluß (23) frei, der mit einem Unterdruckreservoir verbunden ist, das als aufgezogene Einmalspritze (27) ausgebildet sein kann, deren Spritzenstempel (33) in aufgezogener Stellung mittels einer Klammer (36) arretiert ist.

Fig. 1

Die Erfindung betrifft eine Infusionsanordnung mit einer Einrichtung zum Abführen eines Bolusvolumens.

Bei einer Infusion wird Flüssigkeit in den venösen oder arteriellen Teil des menschlichen Kreislaufes zugeführt. Der breite Aufgabenbereich der Infusionstherapie liegt im wesentlichen auf den Gebieten der Zufuhr von Kalorienträgern bei künstlicher Ernährung, Zufuhr von Medikamenten, Regulierung des Elektrolyt-Haushaltes und des Säure-Basen-Gleichgewichtes sowie Flüssigkeitszufuhr zur forcierten Ausscheidung von Giftstoffen durch die Niere, zum Beispiel bei Schlafmittelvergiftung usw..

Innerhalb des Krankenhauses werden Infusionen in den unterschiedlichsten Betriebstellen vorgenommen, sei es in der Intensivmedizin, der Aufnahme und im Notfalldienst, bei der Operation, Entbindung, Säuglings- und Kinderpflege, Funktionsdiagnostik oder allgemein der Normalpflege.

Eine Infusion kann manuell oder unter Einsatz von Infusionsapparaten durchgeführt werden. Für die Wahl der zweckmäßigsten Infusionstechnik sind die geforderte Infusionsrate, die Infusionsdauer, die Dosiergenauigkeit und die erforderliche periphere oder zentrale Applikationstechnik ausschlaggebend.

Die zu applizierenden Medikamente werden immer wirksamer, das heißt die Volumina werden immer kleiner. Dies gilt insbesondere in der Pädiatrie und Neonatologie. Um hochwirksam Medikamente sicher und volumengenau über eine lange Zeiteinheit zu verabreichen, verwendet man heute Infusionspumpen und Infusionsspritzenpumpen. Diese Anwendung ist weit verbreitet und Stand der Technik. Alle Infusionspumpen und Spritzenpumpen haben jedoch die Eigenschaft, Druck zu erzeugen. Dieser Druck wird erst dann wirksam, wenn es beim Patienteneingang, in aller Regel dem Katheter, oder im Infusionsschlauch zum Teilverschluß oder zum Verschluß kommt. Es werden mit modernen Geräten Drucke von ca. 1 bar, mit alten Geräten bis zum 5 bar aufgebaut, bevor die Geräte per Alarm den Verschluß melden.

Ein Infusionshemmnis kann auch einer der vielen Verteilerhähne in den Infusionsleitungen darstellen. So kommt es vor, daß das Bedienungspersonal beim Zusammenstellen der verschiedenen Infusionen vergißt, einen der 3-Wege-Hähne zu öffnen und trotzdem die Infusionspumpe startet.

Bevor eine der parallel geschalteten Infusionspumpen und Spritzenpumpen den ungewollten Verschluß feststellt und alarmiert, baut sich in allen Zuleitungen der genannte erhöhte Druck auf. Verbunden mit dieser Druckerhöhung dehnen sich die Kunststoffleitungen bzw. Schläuche aus und erhöhen dadurch ihr normales Volumen um ca. 2 bis 5 ml je nach Anzahl und Vorspannung der Infusionsleitungen bzw. Infusionspumpen. Dieses gespeicherte Volumen nennt man in der medizinischen Fachsprache "Bolusvolumen".

Im Alarmfall eines der verwendeten Infusionsgeräte muß das Bedienungspersonal den Verschluß beseitigen. Beim Öffnen eines Verschlusses, sei es nur ein 3-Wege-Hahn oder durch Bewegen des Katheters in der Vene, würde dieses Bolusvolumen innerhalb kürzester Zeit in den Patienten strömen und die plötzliche, hohe verabreichte Dosis könnte je nach Medikament dramatische Folgen im Patient auslösen. Die Folgen sind vorstellbar und in Fachkreisen hinreichend bekannt. Da es heute noch keine apparative Warnung vor derartigen Gefahren gibt, ist man auf die Zuverlässigkeit des medizinischen Personals angewiesen.

So muß im Alarmfall das Personal das gesamte System durch Öffnen einer Stelle vom Druck und dadurch vom Bolus befreien, damit der Zugang zum Patienten nicht die Boluswirkung erfährt.

Aus der EP 0 163 373 A1 ist ein 3-Wege-Ventil bekannt, das es ermöglicht, einerseits Infusionsflüssigkeit aus einer Vorratsflasche durch das Ventil dem Patienten zuzuführen, andererseits Infusionsflüssigkeit über eine Spritze durch das Ventil dem Patienten zuzuführen. Das Ventil weist ein von Infusionsflüssigkeit durchströmtes Behältnis auf, wobei ein flaschenseitiger Anschluß des Behältnisses mit einem zur Flasche führenden Schlauch verbunden ist, ein patientenseitiger Anschluß, der gleichzeitig den pumpenseitigen Anschluß darstellt, mit einem patientenseitigen/pumpenseitigen Infusionsschlauch verbunden ist, sowie ein Zuspritzanschluß zum Einführen der Spritze vorgesehen ist. Im Behältnis ist zwischen dem flaschenseitigen Anschluß und dem Zuspritzanschluß ein verschiebbarer Kolben angeordnet, der einen in Strömungsverbindung mit diesen beiden Anschlüssen stehenden Durchgang aufweist. Ein in Richtung des Zuspritzkolbens mittels einer Feder vorgespanntes Absperrelement dichtet den Durchgang ab. Bei nicht beaufschlagtem Spritzenstempel kann Infusionsflüssigkeit aus der Flasche durch den flaschenseitigen Anschluß am Kolben vorbei durch eine zwischen Kolben und Behälterwand befindlichen Ringspalt zum patientenseitigen Anschluß strömen. Der Zuspritzanschluß ist dabei durch den Kolben abgedichtet. Sobald der Spritzenstempel betätigt wird, verursacht der erhöhte Druck eine Verschiebung des Kolbens, so daß dieser im Bereich des flaschenseitigen Anschlusses gegen die Behälterwand gedrückt wird und den unmittelbaren Durchgang vom flaschenseitigen zum patientenseitigen Anschluß sperrt. Gleichzeitig wird durch das Abheben des Kolbens von der dem Spritzenanschluß zugeordneten Behälterwand der Durchgang vom Spritzenanschluß, außen am Kolben vorbei, zum patientenseitigen Anschluß geöffnet. Bei entleerter Spritze hebt die Spitze des Spritzenstempels das Absperrlement

entgegen der Kraft der Feder vom Kolben ab und verschiebt darüber hinaus den Kolben gegen die dem flaschenseitigen Anschluß zugeordnete Behälterwand, so daß bei durch den Spritzenstempel abgedichtetem Zuspritzanschluß ein mittelbarer Strömungsweg der Infusionsflüssigkeit aus der Flasche durch den Ringspalt zwischen Absperrelement und Kolben hindurch zum patientenseitigen Anschluß freigegeben wird. Nach dem Entfernen der Spritze aus dem Behälter drückt die Feder das Absperrelement und diese den Kolben in die den Zuspritzanschluß abdichtende Ausgangslage.

Das beschriebene Ventil dient damit dem Zweck, in der Zuleitung zu einer Infusionspumpe eine Zumischmöglichkeit zu schaffen, ohne den Schwerkraftfluß von der Flasche durch das Ventil zur Infusionspumpe zu blockieren. Es wird außerdem vermieden, daß Infusionsflüssigkeit aus der Flasche in die Spritze läuft. Es ist nicht möglich, daß die Flüssigkeit von der Spritze in die Flasche gedrückt wird. Nach dem Entleeren der Spritze wird automatisch die Zuführung von Infusionsflüssigkeit aus der Flasche herbeigeführt.

Aus der DE 35 03 320 A1 ist ein von Hand verstellbares Mehrwegeventil bekannt, das es ermöglicht, Flüssigkeiten zu Infusaten zuzumischen, bevor diese dem Patienten zugeführt werden. Aus der US 33 86 470 ist ein automatischer 3-Wege-Hahn bekannt, bei dem der anliegende Druck einen Ventilmechanismus steuert. Von einem Einlaß fließt oder drückt Flüssigkeit, an einem vorgespannten Verschlußelement vorbei, in eine Spritze, gleichzeitig wird ein Auslaß mittels eines vorgespannten weiteren Verschlußelementes verschlossen. Beim Betätigen der Spritze wird durch den erhöhten Druck das dem Auslaß zugeordnete Verschlußelement in die geöffnete Stellung überführt und damit der Auslaß freigegeben, während der Einlaß unter Einwirkung einer Feder geschlossen wird.

Es ist Aufgabe der vorliegenden Erfindung, eine Infusionsanordnung mit einer Einrichtung zum Abführen eines Bolusvolumens anzugeben, die es ermöglicht, den ungewollten Bolus automatisch aufzufangen.

Gelöst wird die Aufgabe durch eine Infusionsanordnung mit einer Einrichtung zum Abführen eines Bolusvolumens, die folgende Merkmale aufweist:

- ein von Infusionsflüssigkeit durchströmtes Behältnis,
- einen pumpenseitigen Infusionsschlauch, der mit einem pumpenseitigen Anschluß des Behältnisses verbunden ist,
- einen patientenseitigen Infusionsschlauch, der mit einem patientenseitigen Anschluß des Behältnisses verbunden ist,
- einen mit einem Unterdruckreservoir verbundenen Anschluß,

wobei im Behältnis ein verschiebbares Element angeordnet ist, das einen pumpenseitigen Druckraum vom patientenseitigen Druckraum des Behältnisses trennt und eine Strömungsverbindung der beiden Druckräume vorgesehen ist, und das verschiebbare Element

- in einer Normalstellung den pumpenseitigen Anschluß und den patientenseitigen Anschluß freigibt, sowie den Unterdruckanschluß verschließt,
- in einer Bolusstellung den Unterdruckanschluß freigibt, sowie den patientenseitigen Anschluß verschließt, wobei der pumpenseitige Anschluß frei bleibt,

sowie das verschiebbare Element in Richtung seiner Normalstellung vorgespannt ist.

Im Normalfall, das heißt dann, wenn das verschiebbare Element seine Normalstellung einnimmt, strömt die Infusionsflüssigkeit vom pumpenseitigen Infusionsschlauch in den pumpenseitigen Druckraum des Behältnisses und von dort durch die Strömungsverbindung im verschiebbaren Element zum patientenseitigen Druckraum, um schließlich durch den patientenseitigen Infusionsschlauch dem Patienten zugeführt zu werden. Im Bolusfall wird durch die plötzlich entstehende Druckdifferenz zwischen pumpenseitigem und patientenseitigem Druckraum das verschiebbare Element verschoben, womit der Unterdruckanschluß freigegeben wird und das Bolusvolumen schlagartig in das Unterdruckreservoir aufgesaugt wird. Nach diesem Vorgang wird das in Richtung seiner Normalstellung vorgespannte verschiebbare Element in die Normalstellung zurückbewegt, so daß einerseits die Infusion nur kurzzeitig unterbrochen ist, andererseits der Bolus nicht von selbst in das Infusionssystem zurücklaufen kann, sondern steril im Unterdruckreservoir aufgehoben wird. Nach dem Entfernen des Unterdruckreservoirs kann das aufgefangene Bolusvolumen entweder zurückgegeben oder verworfen werden. Bei Anlegen eines neuen Unterdruckes an das Behältnis ist die Infusionsanordnung mit der Einrichtung zum Abführen des Bolusvolumens wieder einsatzbereit.

Grundsätzlich ist es dabei unabhängig, ob der Verschluß hinter dem Behältnis entsteht und schlagartig abgebaut wird oder ob ein vor dem Behältnis befindlicher Verschluß im Infusionsschlauch schlagartig abgebaut wird und damit die Druckdifferenz auf das Infusionssystem wirkt.

Gemäß einer besonderen Ausführungsform der Erfindung ist vorgesehen, daß das Unterdruckreservoir als mittelbar oder unmittelbar mit dem Unterdruckanschluß verbundene Spritze ausgebildet ist, deren Spritzenkolben nach dem Verbinden der Spritze mit dem Unterdruckanschluß zur Erzeugung eines Unterdruckes aus dem Spritzengehäuse gezogen und in dieser aufgezogenen Stellung arretiert wird. Bevorzugt ist die Spritze als durchsichtige Einmalspritze mit einer Volumenskala ausgebildet. Im Bolusfall verbleibt damit das Bolusvolumen sichtbar und auch meßbar in der Spritze. Ist zusätzlich der durchsichtigen Einmalspritze eine den Füllzustand der Spritze ermittelnde Lichtschranke zugeordnet, kann der Bolusfall auch über eine Signalschaltung angezeigt werden.

Die Verwendung einer Spritze ermöglicht es auf einfache Art und Weise, einen Unterdruck an das Behältnis anzulegen, ferner nach dem Entfernen des Bolus unkompliziert erneut den Unterdruck aufzubringen. Hierzu ist es nur erforderlich, nach dem Verbinden des Spritzenkolbens mit dem Unterdruckanschluß oder einer mit diesem verbundenen Schlauchleitung, den Spritzenkolben aus dem Spritzengehäuse zu bewegen und in einer gewünschten Stellung zu arretieren. In diesem Zusammenhang wird die Verwendung einer Klammer zum Arretieren des Spritzenkolbens zumindest in dessen vollständig aufgezogener Stellung als vorteilhaft angesehen. Die Klammer sollte einen Ansatz am Spritzengehäuse und eine mit der den Spritzenkolben bewegenden Kolbenstange verbundenen Druckplatte in Bewegungsrichtung des Spritzenkolbens formschlüssig umschließen. Die Klammer selbst kann mit mehreren Ausnehmungen zum Arretieren des Spritzenkolbens in unterschiedlichen, aufgezogenen Stellungen versehen sein, so daß variable Unterdrücke aufgebracht werden können. Um sicherzustellen, daß die Klammer nicht verloren geht bzw. um die Infusionsanordnung mit der Einrichtung zum Abführen des Bolusvolumens als bauliche Einheit anwenden zu können, ist es zweckmäßig, wenn ein Klammerhalter vorgesehen ist, der benachbart zum Ansatz am Spritzengehäuse auf dieses aufsteckbar ist und mit dem die Klammer gelenkig verbunden ist. Die Klammer ist so dauerhaft an der Spritze befestigt. Die Klammer und der Klammerhalter sollten zweckmäßig aus Kunststoff bestehen und deren gelenkige Verbindung sollte mittels eines Kunststoffsteges gebildet sein. Die genannten Teile lassen sich damit als Spritzteil herstellen.

Baulich besonders einfach läßt sich die Infusionsanordnung mit der Einrichtung zum Abführen des Bolusvolumens gestalten, wenn das Behältnis relativ zu den pumpenseitigen und patientenseitigen Anschlüssen sowie dem verschiebbaren Element rotationssymmetrisch ausgebildet ist.

Die Strömungsverbindung zwischen den beiden Druckräumen ist vorteilhaft durch eine das verschiebbare Element durchsetzende Durchströmbohrung gebildet. Diese kann sowohl zentrisch als auch exzentrisch im verschiebbaren Element angeordnet sein. Grundsätzlich ist es aber auch möglich, daß die Strömungsverbindung räumlich zwischen dem Behälter und dem verschiebbaren Element erfolgt.

Vorteilhaft ist zwischen dem dem patientenseitigen Anschluß zugeordneten Behälterabschnitt und dem verschiebbaren Element ein dieses in seine Normalstellung vorspannendes Rückstellelement vorgesehen. Dieses kann beispielsweise als Druckfeder, elastisch komprimierbarer Schwamm oder elastisch komprimierbares Kunststoffgitter ausgebildet sein.

Der pumpenseitige Druckraum ist insbesondere dadurch gebildet, daß das Behältnis im Bereich des pumpenseitigen Anschlusses einen Anschlag aufweist, an dem das verschiebbare Element in dessen Normalstellung beabstandet zur Behältereintrittsöffnung des pumpenseitigen Anschlusses anliegt.

Für den Fall, daß die Durchtrittsbohrung das verschiebbare Element exzentrisch durchsetzt, ist vorgesehen, daß der pumpenseitige Anschluß zentrisch in den patientenseitigen Druckraum ragt, wobei das verschiebbare Element in seiner Bolusstellung an der diesem zugewandten Öffnung anliegt und damit den patientenseitigen Anschluß verschließt. Durch diese Verschließmöglichkeit ist sichergestellt, daß im Bolusfall die Infusionsflüssigkeit nicht vom Patienten in das Unterdruckreservoir überströmen kann. Entsprechendes kann ohne ein solches Zusammenwirken von patientenseitigem Anschluß und verschiebbarem Element auch dadurch erzielt werden, daß die Durchströmbohrung ein in der Bolusstellung die Durchströmbohrung in Fließrichtung zum pumpenseitigen Druckraum sperrendes Ventil aufweist. Bevorzugt ist das Ventil mit einem in Fließrichtung zum pumpenseitigen Druckraum vorgespannten Absperrelement versehen, das in der Normalstellung des verschiebbaren Elementes an einem inneren Lageransatz des verschiebbaren Elementes anliegt, wobei eine Strömungsverbindung zwischen dem Absperrelement und dem Lageransatz gewährleistet ist, sowie in der Bolusstellung vom Lageransatz weg gegen einen Ventilsitz gedrückt ist.

In einfachster Ausgestaltung ist das verschiebbare Element als im Behältnis geführter Verschiebekolben ausgebildet. Es versteht sich von selbst, daß insbesondere die wirksamen Druckflächen des pumpenseitigen Druckraumes so zu bemessen ist, daß die durch das Unterdruckreservoir auf den Verschiebekolben ausgeübte Radialkraft dessen Verschiebung nicht entgegensteht. Eine besondere Ausführungsform sieht in diesem Zusammenhang vor, daß der Verschiebekolben im Bereich des pumpenseitigen Druckraumes und des patientenseitigen Druckraumes jeweils eine Abdichtung mittels eines O-Ringes aufweist, wobei zwischen den beiden O-ringen ein Luftpolster gebildet ist, das in der Normalstellung des Verschiebekolbens mit dem Unterdruckanschluß verbunden ist.

Weitere Merkmale der Erfindung sind in der Beschreibung der Figuren dargestellt, wobei bemerkt wird, daß alle Merkmale sowie Kombinationen von Merkmalen erfindungswesentlich sind.

In den Figuren ist die Erfindung an zwei Ausführungsformen dargestellt, ohne auf diese beschränkt zu sein. Es stellt dar:

Figur 1    eine schematische Darstellung der ersten Ausführungsform der Erfindung, gezeigt in der Normalstellung,

Figur 2    eine Darstellung gemäß Figur 1 für den Fall der Bolusstellung,

Figur 3    eine schematische Darstellung der zweiten Ausführungsform der Erfindung, gezeigt in der Normalstellung und

Figur 4    eine Darstellung gemäß Figur 3 für den Fall der Bolusstellung.

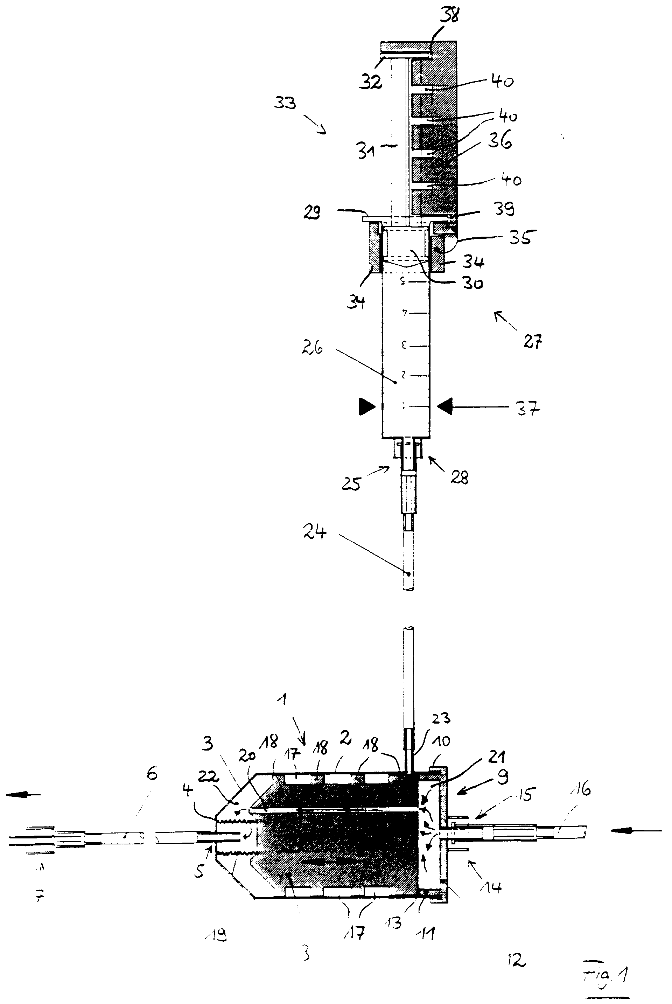

Figur 1 zeigt ein durchsichtiges Kunststoffgehäuse 1, das durch einen zylindrischen Grundkörper 2, einen sich hieran im stumpfen Winkel anschließenden Frontabschnitt 3 und sich eine daran anschließende Frontplatte 4 gebildet ist. Die kreisförmige Frontplatte 4 weist eine zentrische Bohrung auf, in die ein patientenseitiger Anschluß 5 in Form einer Kunststoffhülse eingeklebt ist. Die Kunststoffhülse ragt in das Innere des Gehäuses 1 und steht außen aus diesem hervor. Auf den äußeren Abschnitt des patientenseitigen Anschlusses 5 ist ein durchsichtiger Kunststoffschlauch 6 geklebt, dessen anderes Ende mit einem Luer Lock Male-Konnektor 7 verbunden ist. Dieser Konnektor dient der Verbindung mit einem entsprechenden Konnektor-Female eines nicht weiter gezeigten patientenseitigen Infusionsschlauches.

Innerhalb des Gehäuses 1 ist ein in Aufbau und Funktion noch näher zu beschreibender Verschiebekolben 8 angeordnet. Die patientenferne Öffnung des Gehäuses 1 verschließt ein Deckel 9 aus Kunststoff, der mit dem zylindrischen Grundkörper 2 verklebt ist. Der Deckel 9 weist einen äußeren Flanschring 10 sowie einen inneren Flanschring 11 auf, die zwischen sich das zugewandte Ende des zylindrischen Grundkörpers 2 aufnehmen. Das der eigentlichen Deckelplatte 12 des Deckels 9 abgewandte Ende 13 des Deckels 9 dient als Anschlag für den Verschiebekolben 8. Die Deckelplatte 12 ist mit einer zentrischen Öffnung versehen, mit der ein Luer Lock Konnektor-Female 14 verbunden ist. Mit dem Konnektor 14 ist ein entsprechender Luer Lock Konnektor-Male 15 eines pumpenseitigen Kunststoffschlauches 16 verbunden.

Der Verschiebekolben 8 ist im wesentlichen zylindrisch ausgebildet, mit rillenförmiger Oberfläche, um den Reibungswiderstand zwischen den die Rillen 17 zwischen sich einschließenden umlaufenden Stegen 18 und dem zylindrischen Grundkörper 2 gering zu halten. Die Stege 18 dichten den Verschiebekolben 8 gegenüber dem Gehäuse 1 ab. Das dem patientenseitigen Anschluß 5 zugewandte Ende des Verschiebekolbens 8 ist hinsichtlich seiner Form im wesentlichen entsprechend dem Frontabschnitt 3 und der Frontplatte 4 des Gehäuses 1 ausgebildet. Zwischen der Frontplatte 4 und dem entsprechenden Frontabschnitt des Verschiebekolbens 8 stützt sich zwischen diesem und dem Gehäuse 1 eine Druckfeder 19 ab, die konzentrisch zum patientenseitigen Anschluß 5 angeordnet ist. In der in Figur 1 gezeigten Normalstellung spannt diese Druckfeder 19 den Verschiebekolben 8 gegen das als Anschlag wirkende Ende 13 des Deckels 9 in Richtung des Pfeiles A vor. Die Bewegung des Verschiebekolbens 8 in der entgegengesetzten Richtung gemäß Pfeil B wird durch den patientenseitigen Anschluß 5 begrenzt, sobald der Verschiebekolben 8 den patientenseitigen Anschluß 5 kontaktiert, verschließt er dessen Öffnung. Exzentrisch zur Rotationsachse des Verschiebekolbens 8 durchsetzt diesen eine Durchtrömbohrung 20. Diese verbindet den zwischen dem Deckel 9 und dem Verschiebekolben 8 gebildeten pumpenseitigen Druckraum 21 mit dem zwischen dem Verschiebekolben 8 und der Frontplatte 4, dem Frontabschnitt 3 und dem angrenzenden Bereich des zylindrischen Grundkörpers 2 gebildeten patientenseitigen Druckraum 22.

Der dem Deckel 2 unmittelbar zugewandte Steg 18 des Verschiebekolbens 8 weist gegenüber den anderen Stegen 18 eine größere axiale Erstreckung auf. Im Bereich dieses Steges ist der zylindrische Grundkörper 2 mit einem Unterdruckanschluß 23 in Form einer Kunststoffhülse versehen, auf die ein Kunststoffschlauch 24 aufgesteckt ist. Das gehäuseferne Ende des Kunststoffschlauches 24 ist mit einem Luer Lock Konnektor-Female 25 ausgestattet, auf den das Spritzengehäuse 26 einer Einmalspritze 27 aus Kunststoff mit deren Luer Lock Konnektor-Male 28 aufgeschraubt ist. Die Spritze 27 ist handelsüblich ausgebildet, das heißt deren durchsichtiges Spritzengehäuse 26 weist eine Milliliter-Skaleneinteilung (1 bis 5 ml) auf, ferner ist das schlauchferne Ende des Spritzengehäuses mit einem Griffansatz 29 versehen und in diesem Bereich ist ein Spritzenkolben 30 in das Spritzengehäuse 26 einführbar, der zusammen mit einer Kolbenstange 31 und einer Druckplatte 32 den eigentlichen Spritzenstempel 33 ergibt. Der Spritzenkolben 30 ist vorzugsweise aus elastischem Material, so daß sichergestellt ist, daß er dauerhaft gegenüber dem Inneren des Spritzengehäuses 26 abdichtet. Schließlich umgibt das Spritzengehäuse 26 im Bereich des Griffansatzes 29 in Art eines Aufsteckringes ein Klammerhalter 34 aus Kunststoff, mit dem über einen flexiblen Kunststoffsteg 35 eine in Aufbau und Funktion noch näher zu beschreibende Klammer 36 verbunden ist.

Der Schlauch 24 ist bevorzugt zwischen 5 und 10 cm lang und dient dem Zweck, die Einmalspritze 27 in einem gefälligen Abstand zum Gehäuse 1 zu positionieren. Mit der Bezugsziffer 37 ist schematisch eine Lichtschranke 37 angedeutet, mit der bei vertikaler, nach unten gerichteter Stellung der Spritze 27 ein Füllstand in der Spritze von einem Milliliter erfaßt und signalmäßig dargestellt werden kann.

Nachfolgend sei die Funktion der insoweit beschriebenen Infusionsanordnung mit der Einrichtung zum Abführen des Bolusvolumens beschrieben:

Solange an die Konnektoren 14 und 7 keine pumpenseitige Infusionsleitung oder Sammelleitung bzw. patientenseitige Infusionsleitung angeschlossen ist, befindet sich der Verschiebekolben in der in Figur 1 gezeigten Stellung, das heißt er wird mittels der Druckfeder 19 gegen das als Anschlag dienende Ende 13 des Deckels 9 vorgespannt und verschließt abgedichtet die seitliche Austrittsöffnung zur Einmalspritze 27. Sobald die Infusion angeschlossen ist - oder auch schon bevor der Anschluß der Infusion erfolgt - wird die Spritze 27 aufgezogen, das heißt der Spritzenstempel 33 beispielsweise in die in Figur 1 gezeigte Position bewegt, wobei sich innerhalb des Kunststoffschlauches 24 und des Spritzengehäuses 26 ein Vakuum bildet. Der Spritzenkolben 30 wird dauerhaft in der aufgezogenen Stellung dadurch gehalten, daß die üblicherweise sich radial zum Spritzengehäuse 26 erstreckende Klammer 36 um einen Winkel von etwa 90° in die in Figur 1 gezeigte Position verschwenkt wird und damit die Druckplatte 32 des Spritzenstempels 33 in die oberste Nut 38 der Klammer eingreift. In dieser in der Figur gezeigten Position der Klammer 36 durchsetzt der Griffansatz 29 des Spritzengehäuses 26 eine zurückgesetzte, unterste Nut 39 der Klammer 36, so daß die Klammer 36 durch Eingriff der Druckplatte 32 und des Griffansatzes 29 in diese druck- und zugfest arretiert ist. Zwischen den Nuten 38 und 39 sind entsprechend der Nut 38 gestaltete, weitere Nuten 40 vorgesehen, die ein unterschiedlich weites Aufziehen und anschließendes Arretieren des Spritzenstempels 33 gestatten, so daß ein unterschiedlich hoher Unterdruck einstellbar ist.

In der in Figur 1 gezeigten Normalstellung fließt entsprechend der Pfeildarstellung die Infusionsflüssigkeit durch den pumpenseitigen Schlauch 16 in den pumpenseitigen Druckraum 21, von dort durch die Durchströmbohrung 20 in den patientenseitigen Druckraum 22 und gelangt schließlich durch den patientenseitigen Anschluß 5 und den Schlauch 6 zum Patienten. In dieser Normalstellung ist der Unterdruckanschluß zur Einmalspritze 27 aufgrund der Position des Verschiebekolbens 8 verschlossen.

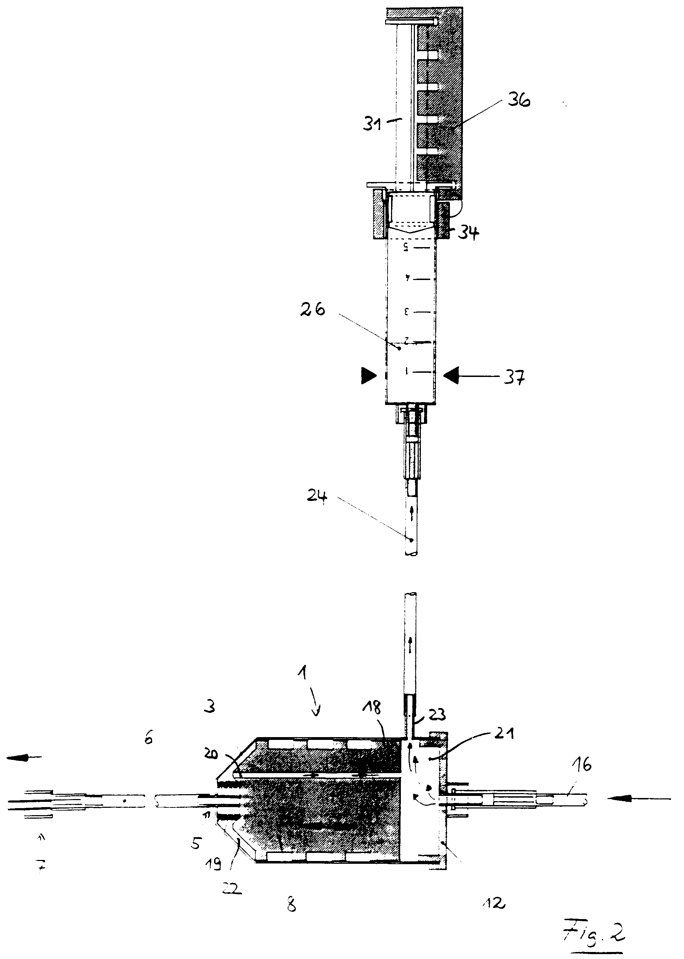

Im Bolusfall, wie er in Figur 2 dargestellt ist, ist der Verschiebekolben 8 infolge der Druckdifferenz zwischen pumpenseitigem Druckraum 21 und patientenseitigem Druckraum 22 in Richtung des Pfeiles B gegen die Kraft der Feder 19 verschoben. In dieser Verschiebestellung, bei der der Verschiebekolben 8 am patientenseitigen Anschluß 5 anliegt und diesen verschließt, ist die Überdeckung des Unterdruckanschlusses 23 durch den Verschiebekolben 8 aufgehoben, so daß das angestaute Bolusvolumen schlagartig infolge des im Spritzengehäuse 26 anstehenden Unterdruckes von der Einmalspritze 27 aufgesogen ist. Infolge der Abdichtung des patientenseitigen Anschlusses 5 über den Verschiebekolben 8 kann während der Abführung des Bolusvolumens keine stromabwärts des Gehäuses 1 befindliche Infusionsflüssigkeit in das Unterdruckreservoir zurückströmen. Nach dem Überströmen des Bolusvolumens in die Einmalspritze 27 bewegt die Druckfeder 19 den Verschiebekolben 8 in Pfeilrichtung A wieder in seine in Figur 1 gezeigte Normalstellung. Figur 2 verdeutlicht, daß ein Bolusvolumen von 2 ml von der Einmalspritze 27 aufgesogen worden ist, was durch die auf 1 ml eingestellte Lichtschranke 37 erkannt und als Signal weitergegeben worden ist.

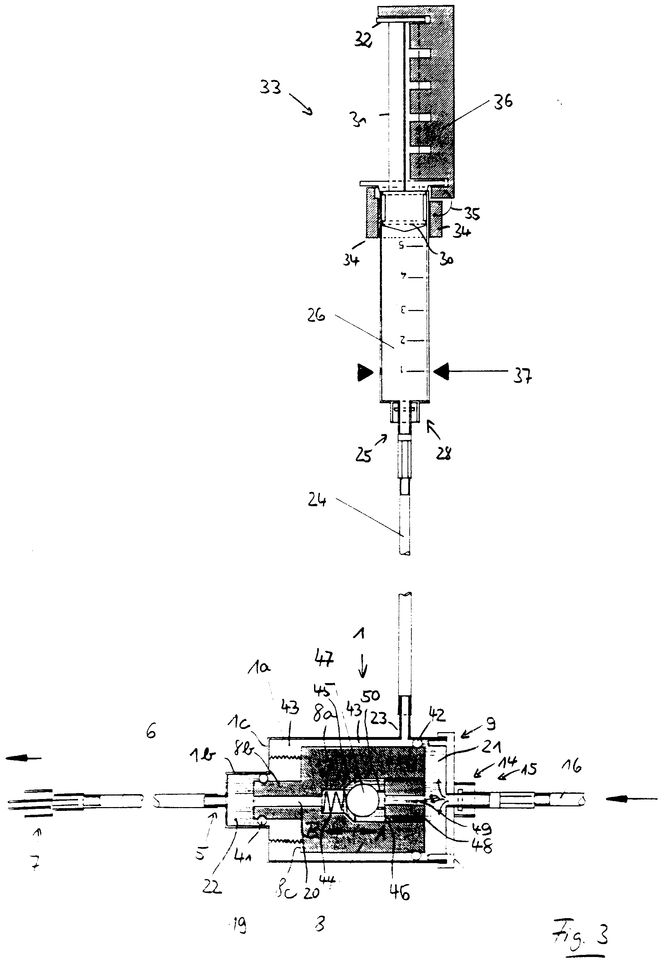

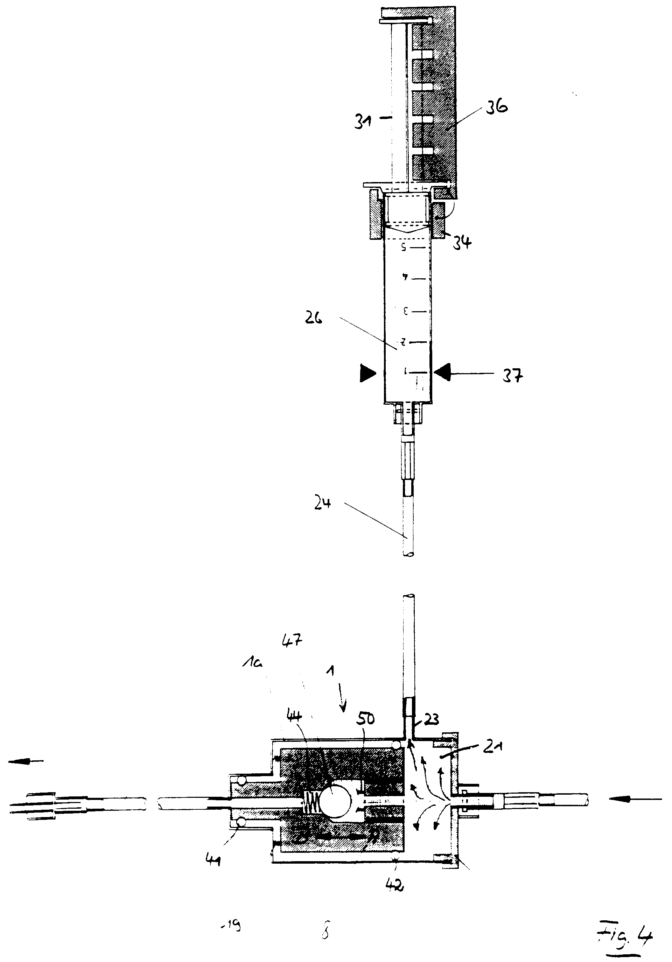

In der modifizierten Ausführungsform nach den Figuren 3 und 4 sind mit der Ausführungsform nach den Figuren 1 und 2 übereinstimmende Bauteile der Einfachheit halber mit gleichen Bezugsziffern bezeichnet.

Die Ausführungsform nach den Figuren 3 und 4 unterscheidet sich von der nach den vorhergehenden Figuren nur durch die besondere Ausbildung des Gehäuses 1 und des Verschiebekolbens 8. Dort ist der Verschiebekolben durch einen zylindrischen Abschnitt 8a großen Durchmessers und einen zylindrischen Abschnitt 8b kleinen Durchmessers gebildet. Entsprechend weist das Gehäuse 1 einen zylindrischen Abschnitt 1a großen Durchmessers und einen zylindrischen Abschnitt 1b kleinen Durchmessers auf. Zwischen der die zylindrischen Abschnitte 8a und 8b verbindenden Ringfläche 8c und der die zylindrischen Abschnitte 1a und 1b verbindenden Ringfläche 1c ist die Druckfeder 19 angeordnet. Der zylindrische Abschnitt 8a des Verschiebekolbens 8 ist mit Spiel im zylindrischen Abschnitt 1a des Gehäuses 1 und der zylindrische Abschnitt 8b mit Spiel im zylindrischen Abschnitt 1b beweglich. Das freie Ende des kleineren zylindrischen Abschnittes 8b des Verschiebekolbens 8 nimmt am Umfang einen O-Ring 41 auf, während das dem Deckel 9 zugewandte Ende des Verschiebekolbens 8 an dessen Umfang einen O-Ring 42 aufnimmt. Die O-Ringe 41 und 42 dichten den Verschiebekolben gegenüber dem zylindrischen Abschnitt 1b bzw. 1a ab, wobei der O-Ring 42 in der in Figur 3 gezeigten Normalstellung sich zwischen dem Deckel 9 und dem Unterdruckanschluß 23 befindet. Bei Aufbringen eines Unterdruckes durch Aufziehen der Einmalspritze 27 (wie in Figur 3 gezeigt) wirkt damit im Ringraum 43 zwischen den beiden O-Ringen 41 und 42 ein Unterdruck.

Wie der Darstellung der Figur 3 zu entnehmen ist, ragt dort der patientenseitige Anschluß 5 nicht in den patientenseitigen Druckraum 22 hinein und ist der Verschiebekolben 8 mit einer zentrischen Durchströmbohrung 20 unterschiedlichen Durchmessers versehen. An einen ersten, dem patientenseitigen Druckraum 22 zugewandten Abschnitt geringen Durchmessers schließt sich ein erweiterter Abschnitt an, der der Aufnahme einer Druckfeder 44 dient, hieran schließt sich ein noch erweiterter Ventilsitzbereich 45 an, der in einen Gewindeabschnitt 46 übergeht. Die diesem zugeordnete Ausnehmung nimmt eine Verschlußkugel 47 auf. In den Gewindeabschnitt 46 ist ein Zylinderelement 48 eingeschraubt, das mit einer Axialbohrung 49 versehen ist. Das Zylinderelement 48 weist auf seiner der Verschlußkugel 47 zugewandten Seite einen Stützkäfig 50 auf, zwischen dem und der Druckfeder 44 die Verschlußkugel 47 beabstandet zum Ventilsitzbereich 45 und zur Axialbohrung 49 in der in Figur 3 gezeigten Normalstellung gehalten ist. In dieser kann damit Infusionsflüssigkeit durch den Kunststoffschlauch 16 den pumpenseitigen Druckraum 21 durch die Axialbohrung 49 zwischen den einzelnen Streben des Stützkäfigs 50 und an der Verschlußkugel 47 sowie der Druckfeder 44 vorbei in den engen Abschnitt der Durchströmbohrung 20 gelangen und von dort durch den patientenseitigen Druckraum 22 und den Kunststoffschlauch 6 zum Patienten.

Sobald, wie in Figur 4 verdeutlicht, der Bolusfall eintritt, bedingt die Druckdifferenz, daß der Verschiebekolben 8 in Richtung des Pfeiles B bewegt wird und der O-Ring 42 hinter den Unterdruckanschluß 23 verschoben wird, so daß das Bolusvolumen in die Einmalspritze 27 entweichen kann. Gleichzeitig wird die Verschlußkugel 47 wegen der Druckdifferenz zwischen dem pumpenseitigen Druckraum 21 und dem patientenseitigen Druckraum 22 vom Stützkäfig 50 abgehoben und entgegen der Kraft der Druckfeder 44 gegen den Ventilsitzbereich 45 gedrückt, so daß ein Rückströmen von Infusionsflüssigkeit von stromabwärts des Gehäuses 1 in den pumpenseitigen Druckraum 21 ausgeschlossen ist. Nachdem das Bolusvolumen abgeführt ist, bewegt die Druckfeder 19 den Verschiebekolben 8 wieder in die in Figur 3 gezeigte Stellung, wegen des gesunkenen Druckes im pumpenseitigen Druckraum 21 wird auch die Verschlußkugel 47 über die Druckfeder 44 wieder gegen den Stützkäfig 50 bewegt.

**Patentansprüche**

1. Infusionsanordnung mit einer Einrichtung zum Abführen eines Bolusvolumens, die folgende Merkmale aufweist:
    - ein von Infusionsflüssigkeit durchströmtes Behältnis (1),
    - einen pumpenseitigen Infusionsschlauch (16), der mit einem pumpenseitigen Anschluß (14) des Behältnisses (1) verbunden ist,
    - einen patientenseitigen Infusionsschlauch (6), der mit einem patientenseitigen Anschluß (5) des Behältnisses (1) verbunden ist,
    - einen mit einem Unterdruckresevoir (33) verbundenen Anschluß (23),
    wobei im Behältnis (1) ein verschiebbares Element (8) angeordnet ist, das einen pumpenseitigen Druckraum (21) von einem patientenseitigen Druckraum (22) des Behältnisses (1) trennt und eine Strömungsverbindung (20) der beiden Druckräume (21, 22) vorgesehen ist und das verschiebbare Element (8)
    - in einer Normalstellung den pumpenseitigen Anschluß (14) und den patientenseitigen Anschluß (5) freigibt sowie den Unterdruckanschluß (23) verschließt,
    - in einer Bolusstellung den Unterdruckanschluß (23) freigibt, sowie den patientenseitigen Anschluß (5) verschließt, wobei der pumpenseitige Anschluß (14) frei bleibt,
    sowie das verschiebbare Element (8) in Richtung seiner Normalstellung vorgespannt ist.

2. Anordnung nach Anspruch 1, bei der das Unterdruckreservoir als mittelbar oder unmittelbar mit dem Unterdruckanschluß (23) verbundene Spritze (33) ausgebildet ist, deren Spritzenkolben (30) nach dem Verbinden der Spritze (33) mit dem Unterdruckanschluß (23) zur Erzeugung eines Unterdruckes aus dem Spritzengehäuse (26) gezogen und in dieser aufgezogenen Stellung arretiert wird.

3. Anordnung nach Anspruch 2, bei der die Spritze (33) über eine Schlauchleitung (24) mit dem Unterdruckanschluß (23) verbunden ist.

4. Anordnung nach Anspruch 2 oder 3, bei der die Spritze als durchsichtige Einmalspritze (33) mit Volumenskala ausgebildet ist.

5. Anordnung nach Anspruch 4, bei der der durchsichtigen Einmalspritze (33) eine den Füllzustand der Spritze (33) ermittelnde Lichtschranke (37) zugeordnet ist.

**6.** Anordnung nach einem der Ansprüche 2 bis 5, bei der eine Klammer (36) zum Arretieren des Spritzenkolbens (30) zumindest in dessen vollständig aufgezogener Stellung vorgesehen ist, wobei die Klammer (36) einen Ansatz (29) am Spritzengehäuse (26) und eine mit der den Spritzenkolben (30) bewegenden Kolbenstange (31) verbundenen Druckplatte (32) in Bewegungsrichtung des Spritzenkolbens (30) formschlüssig umschließt.

**7.** Anordnung nach Anspruch 6, bei der die Klammer (36) mit mehreren Ausnehmungen (40) zum Arretieren des Spritzenkolbens (30) in unterschiedlichen aufgezogenen Stellungen versehen ist.

**8.** Anordnung nach Anspruch 6 oder 7, bei der ein Klammerhalter (34) vorgesehen ist, der benachbart zum Ansatz (29) am Spritzengehäuse (26) auf dieses aufsteckbar ist und mit dem die Klammer (36) gelenkig verbunden ist.

**9.** Anordnung nach Anspruch 8, bei der die Klammer (36) und der Klammerhalter (34) aus Kunststoff bestehen und deren gelenkige Verbindung mittels eines Kunststoffsteges (35) gebildet ist.

**10.** Anordnung nach einem der Ansprüche 1 bis 9, bei der das Behältnis (1) relativ zu den pumpenseitigen und patientenseitigen Anschlüssen (14, 5) sowie dem verschiebbaren Element (8) rotationssymmetrisch ausgebildet ist.

**11.** Anordnung nach einem der Ansprüche 1 bis 10, bei der die die beiden Druckräume (21, 22) des Behältnisses (1) verbindende Strömungsverbindung (20) durch eine das verschiebbare Element (8) durchsetzende Durchströmbohrung (20) gebildet ist.

**12.** Anordnung nach einem der Ansprüche 1 bis 11, bei der zwischen dem dem patientenseitigen Anschluß (5) zugeordneten Behälterabschnitt und dem verschiebbaren Element (8) ein dieses in seiner Normalstellung vorspannendes Rückstellelement (19) vorgesehen ist.

**13.** Anordnung nach Anspruch 12, bei der das Rückstellelement als Druckfeder (19), elastisch komprimierbarer Schwamm oder elastisch komprimierbares Kunststoffgitter ausgebildet ist.

**14.** Anordnung nach einem der Ansprüche 1 bis 13, bei der das Behältnis (1) im Bereich des pumpenseitigen Anschlusses (14) einen Anschlag (13) aufweist, an dem das verschiebbare Element (8) in dessen Normalstellung beabstandet zur Behältereintrittsöffnung des pumpenseitigen Anschlusses (14) anliegt.

**15.** Anordnung nach einem der Ansprüche 1 bis 14, bei der die Durchströmbohrung (20) das verschiebbare Element (8) exzentrisch durchsetzt und der patientenseitige Anschluß (5) zentrisch in den patientenseitigen Druckraum (22) ragt, wobei das verschiebbare Element (8) in seiner Bolusstellung die diesem zugewandte Öffnung des patientenseitigen Anschlusses (5) verschließt.

**16.** Anordnung nach einem der Ansprüche 1 bis 14, bei der die Durchströmbohrung (22) ein in der Bolusstellung die Durchströmbohrung (20) in Fließrichtung zum pumpenseitigen Druckraum (21) sperrendes Ventil (45, 47) aufweist.

**17.** Anordnung nach Anspruch 16, bei der das Ventil (45, 47) ein in Fließrichtung zum pumpenseitigen Druckraum (21) vorgespanntes Absperrelement (47) aufweist, das in der Normalstellung des verschiebbaren Elementes (8) an einem inneren Lageransatz (50) des verschiebbaren Elementes (8) anliegt, wobei eine Strömungsverbindung zwischen dem Absperrelement (47) und dem Lageransatz (50) gewährleistet ist, sowie in der Bolusstellung vom Lageransatz (50) weg gegen einen Ventilsitz (45) gedrückt ist.

**18.** Anordnung nach einem der Ansprüche 1 bis 17, bei der das verschiebbare Element als im Behältnis (1) geführter Verschiebekolben (8) ausgebildet ist.

**19.** Anordnung nach einem der Ansprüche 1 bis 18, bei der der Verschiebekolben (8) im Bereich des pumpenseitigen Druckraumes (21) und des patientenseitigen Druckraumes (22) jeweils eine Abdichtung mittels eines O-Ringes (41, 42) aufweist, wobei zwischen den beiden O-Ringen (41, 42) ein Luftpolster gebildet ist, das in der Normalstellung des Verschiebekolbens (8) mit dem Unterdruckanschluß (23) verbunden ist.

EP 0 489 297 A1

Fig. 1

9

Fig. 2

Fig. 3

Fig. 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | WO-A-8 802 639 (BISCHOF) <br> * Zusammenfassung; Abbildungen 1-4 * <br> --- | 1 | A61M39/00 <br> A61M5/168 |
| A | US-A-4 043 332 (METCALF) <br> * Zusammenfassung; Abbildungen 1,3-5 * <br> --- | 1 | |
| A | FR-A-2 444 472 (ABBOTT LABORATORIES) <br> * Seite 3, Zeile 34 - Seite 4, Zeile 28; Abbildungen 2,3 * <br> ----- | 1 | |
| | | | **RECHERCHIERTE SACHGEBIETE (Int. Cl.5)** <br><br> A61M <br> F16K |

Der vorliegende Recherchenbericht wurde für alle Patentansprüche erstellt

| Recherchenort | Abschlußdatum der Recherche | Prüfer |
|---|---|---|
| DEN HAAG | 13 FEBRUAR 1992 | ZEINSTRA H. |